(19) **Europäisches Patentamt**
**European Patent Office**
**Office européen des brevets**

(11) **EP 1 932 526 A1**

(12) **EUROPEAN PATENT APPLICATION**

(43) Date of publication:
**18.06.2008 Bulletin 2008/25**

(51) Int Cl.:
*A61K 31/352* (2006.01)     *A61P 31/00* (2006.01)
*A61P 31/04* (2006.01)

(21) Application number: **06025629.4**

(22) Date of filing: **12.12.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(71) Applicant: **Leibniz-Institut für Naturstoff-
Forschung
und Infektionsbiologie e.V.
Hans-Knöll-Institut
07745 Jena (DE)**

(72) Inventors:
• **Müller, Peter Jürgen
07745 Jena (DE)**

• **Ozegowski, Jörg-Hermann
07743 Jena (DE)**
• **Peschel, Gundela
07743 Jena (DE)**
• **Presselt, Renate
07768 Kahla (DE)**
• **Siering, Armin
07747 Jena (DE)**

(74) Representative: **Donath, Dirk
Patent- und Rechtsanwälte Bock Bieber Donath
Hans-Knöll-Strasse 1
07745 Jena (DE)**

(54) **Morin, a new inhibitor of microbial hyaluronate lyase**

(57)    This invention relates to a new inhibitor of microbial hyaluronate lyase and its use.

The task of the invention to provide a new inhibitor of microbial hyaluronate lyase and to specify its applications is solved by using the flavonol morin as the inhibitor and by manufacturing pharmaceutical and cosmetic formulations that contain morin.

EP 1 932 526 A1

**Description**

This invention relates to a new inhibitor of microbial hyaluronate lyase and its use.

Microbial hyaluronate lyase is formed by invasive microbial pathogens belonging to the pneumococci, bacteroids, micrococci, peptococci, streptococci, staphylococci, clostridia, propionic bacteria, Yersinia, enterococci, vibriones and Candida, among them a lot of species colonizing skin and mucous membranes, as well as parasites such as Leishmania, Tetrahymena and Trypanosoma. With hyaluronate lyase, they destroy the connective tissue, reduce the barrier effect of tissue boundaries, change the tissue turgor and consequently the conditions for the immigration of immune competent cells and they release proteoglycan fixed endogenic chemotactic modulators in an irregular manner. Therefore, the hyaluronate lyases are an important pathogenicity factor for many infections. As the hyaluronate lyases can remain as an active enzyme in the body even after a successful antimicrobial treatment, their pathogenetic tissue modifying and cell modulating effects on the host are not hampered. As a product of normal microbial skin flora they can also cause an accelerated skin ageing and wrinkling.

The mechanism of hyaluronate lyases principally differs from the hydrolytically acting hyaluronidases that have the effect of β-eliminases. Hyaluronidases are enzymes that hydrolytically cleave the hyaluronic acid biopolymer in combination with a hydrating effect. Therefore, it cannot be expected that the inhibitors of hyaluronidases are necessarily inhibitors of hyaluronate lyases.

In contrast to hyaluronidases, only few inhibitors of microbial hyaluronate lyases are known. An example are synthetic copolymers that are produced by the condensation of formaldehyde and Gentisic acid (H. J. Rogers, S. P. Spensley, Biochim. Biophys. Acta, 1954, 13, 293-297) and inhibit the hyaluronate lyase of *Streptococcus agalactiae.* Sulphated hyaluronic acid is also a good inhibitor (H. Rodig, J. H. Ozegowski, G. Peschel, P. J. Müller, Zbl. Bakt. 1999, 289, 835-843).

JP2000319154 indicates morin apart from other flavonoids as a phototoxic inhibitor in cosmetic compositions or food or beverages. According to JP5148125, morin is used in toothpaste for its antioxidative and stabilizing effects.

The flavonol morin belongs to the group of flavons, the yellow plant pigments. Morin or 2',3,4',5,7-pentahydroxyflavone (CAS registry number: 480-16-0) is gained from wood of different origins, for example from the wood of *Morus alba* (white mulberry), and is commercially available.

It is the object of the present invention to provide a new inhibitor of hyaluronate lyase and to specify its use.

In the invention, this task is solved by a flavonol according to claim 1. Advantageous embodiments are described in the following claims.

This invention is characterised by the surprising finding that with an inhibitor content of $IC_{50}$ = 0.11 μM flavonol morin is a particularly effective inhibitor of microbial hyaluronate lyase and thus it considerably differs from other flavonols and similar substances, because till now and in comparison to other known hyaluronate lyase inhibitors it shows the highest inhibitory activity against the hyaluronate lyase of pathogenic streptococci (among them the *Streptococcus agalactiae* microorganism).

Thanks to its inhibitory effect on microbial hyaluronate lyase, morin is an ideal adjuvans to be used together with antimicrobially acting substances as a component both of pharmaceutical and cosmetic formulations and of medical products to counteract the damages to humans and animals caused by hyaluronate lyases.

The inventive application of morin has the advantage that it shows a very low toxicity with an $LD_{50}$ value of 555 mg/kg. Another advantage is the fact that with $IC_{50}$ = 100 μM morin has approximately 900 times the inhibitory activity of the animal-derived hyaluronidase of cattle testicle the properties of which come close to the endogenic hyaluronidases. Thus, the application of the morin of the inventive formulations does not imply the risk of influencing endogenic hyaluronidases and does not have a toxic effect.

Therefore, morin is particularly well suitable for solving the inventive task because apart from its considerably high inhibitory activity against hyaluronate lyase and its negligible toxicity and effect on endogenic hyaluronidase, it is always available as a vegetable product.

According to this invention, morin is used in liquid formulations in concentrations between 0.0001 mM and 1.0 mM and in solids in concentrations of at least 0.05 g/g solid.

There is a wide range for the use of morin in the manufacture of pharmaceutical formulations or medical and cosmetic products.

According to this invention, morin is used as an adjuvans together with at least one antimicrobial substance and/or an antiparasitic substance and/or an antimicrobial preservative in pharmaceutical or cosmetic formulations or in combination with the material of medical products or distributed in the latter.

A decisive reason for the use of morin as an adjuvans in disinfectants and preservatives is the fact that microorganisms survive due to the development of resistance to disinfectants and preservatives. Thanks to the blockage of the penetration-promoting effect of their hyaluronate lyase and thanks to adherent morin, the ability to penetrate into skin and mucous membrane surfaces is successfully inhibited so that the formation of colonies is considerably reduced.

Preferentially, the preservatives used are the products mentioned by Wallhäußer (K:H: Wallhäußer; Praxis der Sterilisation Desinfektion - Konservierung (Practice of sterilization disinfection - preservation). 5, completely revised edition, published by Georg Thieme Verlag Stuttgart-New York) such as formalin, silver ions, ethanol, phenols, quaternary ammonium salts or iodine. Morin can also be used together with plant-derived substances or parts of such plants or extracts that have an antimicrobial or antiparasitic effect. A very effective plant-derived antimicrobial agent is for example thymol. In humans and animals, the formulations can be used in an intracorporeal manner or they are applied onto the skin or mucous membrane. Thus, they can be introduced by injection or perfusion into tissue in the vascular system or in body cavities or they can be orally administered. Morin is used in solid formations, e.g. as a component or a coating of medical products such as plasters, wound coverings, dressings, textile formations such as orthopaedic bandages, distance tissue, sterile area formations, supporting stockings, catheters, hearing aid elements, cotton buds or hygienic products such as sanitary towels, nappies, tampons, gloves, and condoms.

Morin can be used in disinfectant solutions to be applied onto the skin or mucous membrane, for example to disinfect wounds, hands or areas. In semi-solid or viscous products, morin can be used for example in ointments, creams, lotions, medical and liquid soaps, shampoos or in lubricants. According to this invention, other liquid formulations containing morin have an effect on the mouth and pharynx areas, for example as gargles, pills and bonbons, or they can be inhaled as an aerosol so that their effect reaches up to the lungs.

Embodiment 1

Hyaluronate lyase of *Streptococcus pyogenes* (Hyal A) is obtained according to Gerlach et al. (Gerlach D, W. Köhler, Zentralbl Bakteriol 221, 166-172, 1972).

Hyaluronate lyase of *Streptococcus equisimilis* (Hyal C) and Hyaluronate lyase of *Streptococcus agalactiae* strain 4755 (Hyal B) are got according to Ozegowski et al (Zentralbl. Bakteriol., 249, 310-318, 1981, Zentralbl. Bakteriol 280, 497-506, 1994).

Hyaluronate lyase of *Streptomyces hyalurolyticus* (Hyal S) and hyaluronidase of cattle testicles are purchased (Sigma Taufkirchen, Germany).

The hyaluronate lyase activity (units/ml) is determined according to Rodig et al. (Zentralbl. Bakteriol 289, 1999, 835-843).

The inhibitory activity (%) is found out in a modified method according to Hertel et al. (Arch. Pharm 339 313-8, 2006) in microtiter plates.

For determining these values, the hyaluronic acid and the enzyme are dissolved in a 0.1 M acetate buffer of pH 6.0. Morin is dissolved as a solid in dimethyl sulphoxide in a concentration of 20 mM and afterwards diluted with water to the corresponding test concentration.

To determine the inhibitory activity, 50 $\mu$l of a hyaluronate lyase solution containing 4 U/ml are given into each well. Then, 10 $\mu$l of the inhibitor solution are added so that the final concentration of morin is between 0.0001 mM and 1.5 mM in the preparation.

After an incubation time of 10 minutes, 50 $\mu$l of hyaluronic acid of 20 mg/100 ml are added and after an incubation of 30 minutes at 37°C the reaction is stopped by the addition of 200 $\mu$l of N-Cetyl-N,N,N-trimethyl ammonium bromide (2.5% w/v in 2% w/v NaOH). The residual cloudiness appearing is measured at 600 nm and the inhibitory activity is calculated according to the following equation:

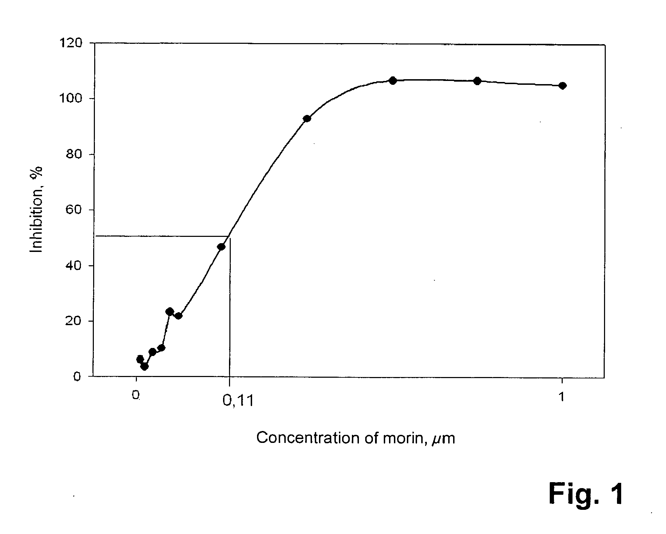

$$\text{Inhibitory activity[\%]} = ((A_{\text{Control}} - A_{\text{Sample}}) \times 100\%) / (A_{\text{Control}}). \text{ (Fig. 1)}$$

Embodiment 2

A recipe for a shampoo that fights dandruff contains 50% alkyl ether sulphate, 28 per cent, 3.0% protein hydrolyzate, 50 per cent, 3.0% diethylene glycol monolauryl ether, 1.0% pirocton olamin, 0.5% morin and water ad 100.

Embodiment 3

A peeling cream for bad skin contains 9.6% cetyl stearyl alcohol, 4% cetyl stearyl alcohol/natrium cetyl stearyl sulphate, 12% low-viscosity paraffin, 0.1% 4-hydroxybenzoic acid ethyl ester, 52.5% water, 5.0% glycerine, 1.0% morin, 0.1% 4-hydroxybenzoic acid ethyl ester, 0.3% hydroxybenzoic acid ethyl ester, 15%, polythene powder and 0.4% phenoxyethanol.

Embodiment 4

A foot powder contains 86.5% French chalk, 7.5% aluminium hydrochloride, 3% silicon dioxide, 2.0% salicylic acid and 1.0% morin.

Embodiment 5

An antiperspirant contains 97.3% isopropanol, 1.0% glycerine, 0.5% farnesol, 0.8% perfume oil, 0.2% morin and 0.2% polysorbate.

Embodiment 6

An O/W cream for the care of bad skin contains 5.0% sulphur, 5.0% middle-chain triglycerides, 19.0% lauric acid hexyl ester, 15% cetyl stearyl alcohol/sodium cetyl stearyl sulphate, 1.0% morin, 0.2% methyl p-hydroxybenzoate, 0.2% triclosan, 0.2% allantoin, 54.3% water and 0.1 % perfume oil.

**Claims**

1.    Inhibitor of microbial hyaluronate lyase in form of the flavonol morin.

2.    Inhibitor as set forth in claim 1, wherein the inhibitor constant $IC_{50}$ of morin is 0.11 $\mu$m.

3.    Use of the inhibitor as set forth in claim 1 or 2 for the manufacture of pharmaceutical formulations or medical or cosmetic products that furthermore contain at least an antimicrobial substance and/or an antimicrobial agent and/or an antiparasitic agent.

4.    Use of the inhibitor as set forth in claim 3, wherein the concentration of morin is between 0.0001 mM and 1.0 mM.

5.    Use of the inhibitor as set forth in claim 3, wherein the concentration of morin in solids is at least 0.05 g/g solid.

6.    Use of the inhibitor as set forth in claim 4 or 5, wherein morin is contained together with an antibiotic and/or a sulfamide.

7.    Use of the inhibitor as set forth in claim 4 or 5, wherein morin is contained together with at least one preservative.

8.    Use of the inhibitor as set forth in claim 4 or 5, wherein morin is contained together with silver or silver ions.

9.    Use of the inhibitor as set forth in claim 4 or 5, wherein morin is contained together with plant-derived substances or vegetable extracts or plant parts that have an antimicrobial or antiparasitic effect.

10.    Use of the inhibitor as set forth in claim 4 or 5, wherein morin is contained together with thymol.

11.    Use of the inhibitor as set forth in claim 4 or 5, wherein morin is contained together with animal-derived substances that have an antimicrobial or antiparasitic effect.

Concentration of morin, $\mu m$

**Fig. 1**

**European Patent Office**

# EUROPEAN SEARCH REPORT

Application Number

EP 06 02 5629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAUHA J-P ET AL: "ANTIMICROBIAL EFFECTS OF FINNISH PLANT EXTRACTS CONTAINING FLAVONOIDS AND OTHER PHENOLIC COMPOUNDS" INTERNATIONAL JOURNAL OF FOOD MICROBIOLOGY, ELSEVIER SCIENCE PUBLISHERS, AMSTERDAM, NL, vol. 56, 25 May 2000 (2000-05-25), pages 3-12, XP002909973 ISSN: 0168-1605 * table 3 * | 1-11 | INV. A61K31/352 A61P31/00 A61P31/04 |
| X | ARIMA HIDETOSHI ET AL: "Rutin-enhanced antibacterial activities of flavonoids against Bacillus cereus and Salmonella enteritidis" BIOSCIENCE BIOTECHNOLOGY AND BIOCHEMISTRY, vol. 66, no. 5, May 2002 (2002-05), pages 1009-1014, XP007901751 ISSN: 0916-8451 * figures 2-7; tables 2,3 * | 1-11 | |
| X | DATABASE WPI Week 200377 Derwent Publications Ltd., London, GB; AN 2003-819104 XP002432472 & JP 2003 171274 A (ALPS YAKUHIN KOGYO KK) 17 June 2003 (2003-06-17) * abstract * | 1-11 | TECHNICAL FIELDS SEARCHED (IPC)<br><br>A61K<br>A61P |
| X | DATABASE WPI Week 200162 Derwent Publications Ltd., London, GB; AN 2001-555269 XP002432473 & KR 2001 0001476 A (LG CHEM CO LTD) 5 January 2001 (2001-01-05) * abstract * | 1-11 | |

The present search report has been drawn up for all claims

| Place of search | Date of completion of the search | Examiner |
|---|---|---|
| Munich | 12 June 2007 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 02 5629

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2007

| Patent document cited in search report | Publication date | Patent family member(s) | Publication date |
|---|---|---|---|
| JP 2003171274 A | 17-06-2003 | NONE | |
| KR 20010001476 A | 05-01-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000319154 B

- JP 5148125 B

**Non-patent literature cited in the description**

- **H. J. ROGERS ; S. P. SPENSLEY.** *Biochim. Biophys. Acta,* 1954, vol. 13, 293-297
- **H. RODIG ; J. H. OZEGOWSKI ; G. PESCHEL ; P. J. MÜLLER.** *Zbl. Bakt.,* 1999, vol. 289, 835-843
- Praxis der Sterilisation Desinfektion - Konservierung. **K:H: WALLHÄUßER.** Practice of sterilization disinfection - preservation. Georg Thieme Verlag

- **GERLACH D ; W. KÖHLER.** *Zentralbl Bakteriol,* 1972, vol. 221, 166-172
- **OZEGOWSKI et al.** *Zentralbl. Bakteriol.,* 1981, vol. 249, 310-318
- *Zentralbl. Bakteriol,* 1994, vol. 280, 497-506
- **RODIG et al.** *Zentralbl. Bakteriol,* 1999, vol. 289, 835-843
- **HERTEL et al.** *Arch. Pharm,* 2006, vol. 339, 313-8